# EUROPEAN PATENT SPECIFICATION

(11) **EP 4 467 974 B1**
(45) Date of publication and mention of the grant of the patent: **30.09.2026**
(21) Application number: 23175726.1
(22) Date of filing: 26.05.2023
(51) Int. Cl.: G01N 27/327, G01N 27/48, G01N 33/94, G01N 33/543

(54) **NOVEL CHEMOSENSORS AND THEIR USE IN ELECTROCHEMICAL DETECTION METHODS**
NEUE CHEMOSENSOREN UND IHRE VERWENDUNG IN ELEKTROCHEMISCHEN NACHWEISVERFAHREN
NOUVEAUX CAPTEURS CHIMIQUES ET LEUR UTILISATION DANS DES PROCÉDÉS DE DÉTECTION ÉLECTROCHIMIQUE

(43) Date of publication of application: 27.11.2024
(73) Proprietor: Karlsruher Institut für Technologie, 76131 Karlsruhe (DE)
(72) Inventor: PICCHETTI, Pierre, 76131 Karlsruhe (DE); MANOJ KUMAR, Nilima, 76131 Karlsruhe (DE); GRUHS, Patrick, 76131 Karlsruhe (DE); BIEDERMANN, Frank, 76131 Karlsruhe (DE)
(74) Representative: Gille Hrabal Partnerschaftsgesellschaft mbB Patentanwälte

(56) References cited:
- LUO HUAN ET AL: "Applications of macrocyclic compounds for electrochemical sensors to improve selectivity and sensitivity", JOURNAL OF INCLUSION PHENOMENA AND MACROCYCLIC CHEMISTRY, KLUWER, DORDRECHT, NL, vol. 95, no. 3-4, 10 July 2019 (2019-07-10), pages 171 - 198, XP036937908, ISSN: 1388-3127, [retrieved on 20190710], DOI: 10.1007/S10847-019-00934-6
- KRÄMER JOANA ET AL: "Molecular Probes, Chemosensors, and Nanosensors for Optical Detection of Biorelevant Molecules and Ions in Aqueous Media and Biofluids", vol. 122, no. 3, 7 January 2022 (2022-01-07), US, pages 3459 - 3636, XP093024061, ISSN: 0009-2665, Retrieved from the Internet <URL:https://pubs.acs.org/doi/pdf/10.1021/acs.chemrev.1c00746> DOI: 10.1021/acs.chemrev.1c00746
- JIANG CHENRUI ET AL: "Fluorescent probes based on macrocyclic hosts: Construction, mechanism and analytical applications", TRAC TRENDS IN ANALYTICAL CHEMISTRY, ELSEVIER, AMSTERDAM, NL, vol. 133, 21 October 2020 (2020-10-21), XP086396231, ISSN: 0165-9936, [retrieved on 20201021], DOI: 10.1016/J.TRAC.2020.116086
- MUTIHAC RADU-CRISTIAN ET AL: "A brief overview on supramolecular analytical chemistry of cucurbit[]urils and hemicucurbit[n]urils", JOURNAL OF INCLUSION PHENOMENA AND MACROCYCLIC CHEMISTRY, KLUWER, DORDRECHT, NL, vol. 98, no. 3-4, 13 August 2020 (2020-08-13), pages 137 - 148, XP037278490, ISSN: 1388-3127, [retrieved on 20200813], DOI: 10.1007/S10847-020-01019-5

## Description

### INTRODUCTION

The invention relates to novel chemosensors comprising a metal-based indicator and a macrocyclic receptor and their use in analytical detection methods for electro-inactive compounds in an Indicator Displacement Assay (IDA).

### BACKGROUND OF THE INVENTION AND PRIOR ART

Current analytical methods for detecting and quantifying electro-inactive compounds in biofluids and liquid medical preparations or administration forms, rely on complex, time-consuming, and costly separation techniques, such as high-performance liquid chromatography (HPLC) combined with mass spectrometry. HPLC-based assays for the detection of analytes involve laborious sample pre-treatment and relatively long assay times. In addition, these analysis techniques have limited potential to be miniaturized. Thus, their implementation in commercial sensors for home use or wearable sensors is a long-term vision with uncertain success.

Such novel analytical test systems are in particular of high interest for the analysis of electro-inactive compounds from the group of drugs, biomolecules and antimicrobial agents. As a specific class of electro-inactive compounds quaternary ammonium compounds are to be mentioned of particular relevance. As a particular example from the group of quaternary ammonium compounds drugs like pancuronium bromide (PB), which act as muscle relaxants, can be mentioned. Pancuronium bromide drugs are broadly used in clinics for general anaesthesia. However, their extensive use is not without controversy, in particular, as improper and unauthorized administration has led to cases of non-functional anaesthesia and the drug has been used by criminals to immobilize their victims. Chromatography and mass spectrometric methods are not an option for the rapid detection of misuse of such a class of compounds for the reasons stated above. Therefore, molecular diagnostic tools for the detection and quantification of such electro-inactive compounds, including such quaternary ammonium drug compounds, must be simple, inexpensive, and rapid to perform so that they can be used at the point of care and operated by non-specialists with minimal sample pre-treatment. Indeed, such sensors could revolutionize drug detection and advance personalized medicine and wearable sensor technologies. The development of a rapid, easy-to-use, miniaturized, and low-cost sensor that can be used at the point of care, i.e., at home, in clinics, or in crime scenes and law enforcement for analysing electro-inactive compounds, such as drugs like pancuronium bromide etc., is of high interest.

Electrochemical sensors (also called "chemosensor(s)") have emerged as a powerful tool for developing miniaturized, disposable, and portable molecular diagnostic instruments with relatively inexpensive components.

Generally, chemosensors are macrocyclic molecules that produce a recordable signal after binding to an analyte. In this context, host-guest type chemosensors are considered promising candidates to accomplish the above-mentioned ambitious goals. At their most basic, chemosensors are macrocyclic molecules (also designated as "receptors" or "host(s)"), which act in combination with an indicator (also designated as "dye") or as a host-dye complex, and produce a spectroscopic readout, such as a change in absorbance or fluorescence, after binding to the analyte (also designated as "guest").

Known macrocyclic receptors with the capability to bind small organic molecules in aqueous media include e.g. naphtotubes, cavitands, calix[n]arenes, as well as cyclodextrin-, cucurbit[n]uril- and pillar*[n]*arene-based macrocyclic receptors.

Cyclodextrin-based chemosensors are available that apply the principles of an IDA for the electrochemical detection of electro-inactive analytes, such as cortisol and cinchonine. However, cyclodextrins are suboptimal because their binding affinity for many biomolecules and drugs is too weak to achieve relevant detection limits. For example, quaternary ammonium compounds, like well-known drugs such as muscle relaxants, cannot be detected at low micromolar, i.e. biologically relevant concentrations using cyclodextrins. In addition, cyclodextrins are receptors that tend to aggregate in solution, e.g., triggered by temperature changes, rendering their use suboptimal for electrochemical sensors. Therewith, cyclodextrin-based methods are far from applicable to aqueous solutions and biofluids because to date such methods require chemical immobilization processes of the cyclodextrin-based chemosensors on the surface of the electrodes or other parts of the sensor device. Such surface immobilizations are expensive and complex to implement and difficult to reproduce, all factors that are detrimental to the development of commercial sensors based on cyclodextrin receptors. Better receptors that are also compatible with IDA formats are needed.

Host-guest type bi- and unimolecular chemosensors are considered promising candidates for the development of simple and low-cost sensors and among the above-mentioned macrocyclic receptors, in particular cucurbit*[n]*uril- and pillar*[n]*arene-based receptors have been identified as suitable. Cucurbit*[n]*urils (hereinafter also designated as "cucurbiturils", also abbreviated as "CBn") exhibit excellent binding affinity for many biomolecules and drugs, including quaternary ammonium compounds. In addition, they are chemically robust and biocompatible, and their synthesis is inexpensive. Pillar*[n]*arene based receptors (hereinafter also designated as "pillararenes") are macrocyclic receptors consisting of hydroquinone or dialkoxybenzene and are structurally similar to cucurbit*[n]*urils.

By the virtue of their binding affinities, cucurbiturils and pillararenes are prominent receptors of fluorescence-based chemosensor assays, including so-called indicator displacement assays (IDA), that are compatible with biofluids, and they are therefore commonly used in fluorescence-based indicator displacement assays for the optical detection of drugs and biomolecules.

However, while useful, fluorescence as the signal output can be a disadvantage if the sample being analyzed contains other fluorescent components or larger particles that scatter light, which results in a suboptimal signal-to-noise ratio. In addition, miniaturization of the optical components that are required to produce and record the fluorescence of chemosensors is expensive and still difficult to accomplish.

For example, cucurbit[7]uril (CB7) has been described as a receptor used in fluorescence-based chemosensor assays in biofluids, e.g. in the form of an indicator displacement assay (IDA). However, for the reasons mentioned above a fluorescent signal readout is disadvantageous for analyzing samples containing other fluorescent components or larger particles that scatter light, resulting in a suboptimal signal-to-noise ratio.

Macrocyclic receptors have been used as recognition elements to accumulate electroactive analytes on the surface of electrodes, thereby contributing to an improved signal-to-noise ratio of electrochemical sensors. Such strategies are useful if the analyte can be electrochemically oxidized or reduced within the electrochemical window of water. However, to detect electrochemically inactive analytes by using chemosensors, other design strategies are required.

In voltammetric or amperometric sensors, the measured signal is a current resulting from direct or indirect electrochemical oxidation or reduction of analytes close to the surface of the working electrode. To date, chemosensors for voltammetric detection of analytes require the use of custom electrodes that are not commercially available. As a result, each chemosensor reported in the past required a specific protocol for manipulating the electrodes before the chemosensor could be immobilized on their surface. These procedures involve complex and costly fabrication steps. As a result, the reproducibility of such electrodes and thus their reliability are low. All in all, the use of such customized electrodes complicates the successful use of supramolecular chemosensors in diagnostic devices. Screen-printed electrodes (SPEs) are commercially available that require minimal sample volume and are compatible with biofluids and aqueous solutions. However, no chemosensor has been successfully used with such SPEs.

The commercial success of electrochemical sensors is reflected in their good signal response time to sensitivity ratio and their low-cost components, with commercially available screen-printed electrodes available for sensing purposes that can be further functionalized according to specific needs. Therefore, the development of new chemosensors that are compatible with commercially available SPEs is of great importance.

The electrochemical properties of organic molecules can be modulated when they form an inclusion complex with macrocyclic receptors. For example, it has been shown that the redox current peak for organic molecules decreases significantly when they form an inclusion complex with a macrocyclic receptor, because the macrocyclic host can be considered as a protective shell surrounding the guest, inhibiting and/or altering the electrochemical processes or properties of the guest [Ong, W.; Kaifer, A. E., Unusual Electrochemical Properties of the Inclusion Complexes of Ferrocenium and Cobaltocenium with Cucurbit[7]uril. Organometallics 2003, 22 (21), 4181-4183]*.* For example, it has been described that when ferrocene (Fc) forms an inclusion complex with CB7 (CB7⊃Fc), lower current levels are observed in voltammetric experiments, which can be attributed to a decreased effective diffusion coefficient of CB7⊃Fc compared to Fc. In addition, a complexation-induced shift in the half-wave potential value for Fc when complexed by CB7 was reported [Jeon, W. S. et al., Complexation of Ferrocene Derivatives by the Cucurbit[7]uril Host: A Comparative Study of the Cucurbituril and Cyclodextrin Host Families. J. Am. Chem. Soc. 2005, 127 (37), 12984-12989*]*.

As for the competitive guest-displacement from macrocyclic receptors that can be followed electrochemically, it has been described as a conceptual example that the oxidation potential of Fc that is immobilized on the surface of gold electrodes (Fc@surface) is shifted to more positive values when an inclusion complex with CB7 is formed (CB7⊃Fc@surface). By direct cyclic voltammetry (CV), it was shown that the potential shift is indicative of the amount of CB7⊃Fc@surface formed in the presence of a competing guest. However, the need for a meticulously uniform self-assembled monolayer on gold electrode surfaces is a major drawback for the development of novel electrochemical chemosensors (e-CS), because the preparation of monolayers is not straightforward, and they have limited electrochemical stability in aqueous solution. Furthermore, the presence of a monolayer on the electrode surface can lead to nonspecific adsorption of analytes [Qi, L.; Wang, R.; Yu, H. Z., Electrochemical Quantitation of Supramolecular Excipient@Drug Complexation: A General Assay Strategy Based on Competitive Host Binding with Surface Immobilized Redox Guest. Anal. Chem. 2020, 92 (2), 2168-2175*;* Beulen, M. W. J. et al., Electrochemical Stability of Self Assembled Monolayers on Gold. Langmuir 1998, 14 (26), 7463-7467].

It is well known that cucurbit*[n]*uril and pillar*[n]*arenes have a high binding affinity to quaternary ammonium compounds. Cucurbit[7]uril has already been used with ferrocene to define the binding affinity of electro-inactive analytes.

The use of cucurbit[8]uril (CB8) in combination with electrochemical sensing has been described, wherein CB8 was immobilized on the surface of modified electrodes and used to detect the amino acid tryptophan [María del Pozo et al., The use of cucurbit[8]uril host-guest interactions in the development of an electrochemical sensor: characterization and application to tryptophan determination; J. Mater. Chem. 2011, 21, 13657; DOI: 10.1039/C1JM12063H*].* Therein, the chemosensor did not function according to the principles of an indicator displacement assay (IDA), but the CB8 serves as a container to capture and accumulate the tryptophan on the surface of the electrode, where tryptophan is directly oxidized electrochemically.

Another electrochemical detection method using CB8-immobilized electrodes has been described to measure dopamine, which is an electroactive analyte [María del Pozo et al., Cucurbit[8]uril-based electrochemical sensors as detectors in flow injection analysis. Application to dopamine determination in serum samples; Sens. Actuators B Chem. 2014, 193, 62; DOI: 10.1016/j.snb.2013.11.074]. The method described therein also does not operate in an IDA-based format.

It has further been described to use modified electrodes on which a cucurbit[7]uril (CB7)-methylene blue host-guest complex is immobilized to detect enzymatic activities. Therein, peptide fragments that form in the presence of enzymes bind to CB7 and simultaneously displace methylene blue. The free methylene blue is detected electrochemically, and the measured current is an indicator of the presence of the enzymes and allows qualitative and quantitative analysis [Wang et al., An electrochemical peptide cleavage-based biosensor for matrix metalloproteinase-2 detection with exonuclease III-assisted cycling signal amplification, Sens. Actuators B Chem. 2014, 193, 62; DOI: 10.1016/jsnb.2013.11.074]. Although this electrochemical sensor follows the principles of an IDA, therein immobilization on electrodes is required.

A further method describes the use of a CB7-based monolayer prepared on gold electrodes, where the immobilized CB7 can bind ferrocene (Fc). Therein it is described that the ferrocene is immobilized on the electrode surface via binding to the CB7 monolayer and therefrom is displaced by the presence of the compounds denatonium benzoate (DB), alagebeium (ALA), or adamantanol (AdOH). The displacement was monitored by oxidation of free Fc at the electrode. This displacement of ferrocene was used to determine the binding affinity of the above compounds to CB7 [Qi et al., Electrochemical Quantitation of Supramolecular Excipient@Drug Complexation: A General Assay Strategy Based on Competitive Host Binding with Surface-Immobilized Redox Guest Anal. Chem. 2020, 92, 2168; DOI: 10.1021/acs.analchem.9b04803]*.* The method described therein was not used for sensing purposes but relates to the thermodynamic characterization of the binding of ferrocene as the indicator on CB7. The method described therein requires the immobilization of the receptor and the ferrocene on the electrode surface.

A similar approach is described in CN000113671002A, which relates to a method for detecting bioactive substances based on gold surface modified cucurbit[7]uril.

Further, low-cost and high-performance acetylcholine electronic sensors based on ionselective electrodes (ISEs) functionalized with cucurbit[6]uril (CB6) derivatives have been described, wherein a membrane in which CB6 was immobilized, rather than an electrode, was used to detect acetylcholine. Therein, the membrane forms a functional element of the ISE device and the CB6 simply traps the analyte, which in turn changes the ion activity in the solution that can be detected by the ISE in a potentiometric analysis [Zhao et al., Cucurbit[n]uril Derivatives Soluble in Water and Organic Solvents; Angew. Chem. Int. Ed. 2001, 40, 4233; DOI: 10.1002/1521-3773(20011119)40:22<4233:AID-ANIE4233>3.0.CO;2-D].

As for cucurbit*[n]*urils, also the use of pillararenes as receptors / chemosensors in electrochemical detection methods has been described, however, only by immobilization thereof on electrode surfaces.luo
The so far described methods using IDA-based chemosensors with electrochemical readout require the use of cyclodextrins with the disadvantages described above, and/or they require modification of electrode surfaces and/or surface immobilization of chemosensors on electrodes or other parts of the devices, making them less suitable for rapid, easy-to-use, miniaturized, and low-cost sensors that can be used at the point of care for the desired applications described above.

### OBJECT OF THE INVENTION

It was the object of the invention to provide methods for detecting electro-inactive compounds, such as quaternary ammonium compounds, which avoid the above discussed disadvantages. The new method should be able to circumvent the drawbacks of optical-based IDAs and existing IDAs with an electrochemical signal readout for the detection and quantification of biologically relevant analytes. The methods should, in particular, avoid fluorescence-based detection methods. The methods should be applicable in aqueous test samples, in particular, directly in biofluids and samples derived from body fluids. The method should not require the use of cyclodextrins as chemosensors, which are suboptimal for the detection of bioanalytes or drugs but should be based on robust and stable chemosensors being applicable in biofluid-samples. The method should be broadly applicable and easy to prepare and should not require modification of electrode surfaces and avoid the problematic surface immobilization of chemosensors on electrodes or other parts. The method should be applicable by using commercially available electrodes and detection equipment to allow low production costs, to offer easy-to-use chemosensor design with electrical signal readout and ideally allow the use of readily available screen-printed electrodes.

### SUMMARY OF THE INVENTION

To avoid the above-mentioned disadvantages of the prior art and solve the objects described above, the inventors of the present invention developed an electrochemical detection method for the detection and quantification of electro-inactive compounds, like quaternary ammonium compounds, using cucurbit*[n]*uril or pillar*[n]*arene based bi- and unimolecular IDA (Indicator Displacement Assays)-based chemosensors with metal-based indicators.

The method is based on trapping a metal-based indicator, e.g. in the form of a metal-based complex, in the cavity of the receptor. When the analytes (e.g. quaternary ammonium compounds) to be detected displace the indicator in the cavity of the receptor, it can be electrochemically oxidized, which can be measured via voltammetry or amperometry.

With the method of the present invention, it is possible to provide reliable, rapid and costeffective methods for the detection of electro-inactive compounds, such as quaternary ammonium compounds, offering new options for detecting e.g. drugs in biofluids. The receptors of the invention enable a wider range of analytes that can be detected at biologically relevant concentrations when compared to previously used cyclodextrin receptors.

The invention provides an assay using bi- or unimolecular chemosensors based on cucurbit*[n]*urils and pillararenes or derivatives thereof with a variety of electrochemically active indicators, such as a variety of metal-based indicators. The chemosensors as described herein are water-soluble and form stable solutions in buffers and biofluids, e.g. urine. Further, the chemosensors described herein are not adsorbed onto carbon screen-printed electrodes. The chemosensors of the invention can be used with various electrochemically active and metal-based indicators.

The method and the assay of the invention offers the advantages that a minimal number of sample manipulation steps is required, because the chemosensor can directly be assembled in the sample, i.e. body fluids. Further, the samples to be analysed, e.g. buffers or body fluids like urine, need no purification or separation procedures before analysis. The working procedure using the assay of the invention is user-friendly in that it can be easily operated by untrained personnel, is inexpensive, quick to perform, requires minimal sample volumes, and has the required sensitivity for biomedical applications, including e.g. detection of drug abuse. A further advantage is that the chemosensors of the present invention do not need to be immobilized on the surface of electrodes, nanomaterials, or any other part of the testing devices. In contrast, they can be applied (e.g. dropped) directly to commercially available screen-printed electrodes without manipulating the electrodes, which makes them compatible with commercially available screen-printed electrodes (SPEs). The method and assay is suitable to be implemented and provided in the form of miniaturized and supramolecular chemosensor devices, making them suitable as point-of-care diagnostics and portable sensors. Therewith the new methods can easily be used at a broad variety of settings, including home-use, in clinics, etc. or as wearable (portable) sensors.

The present invention is described in more detail as follows.

### DETAILED DESCRIPTION OF THE INVENTION

In a first aspect the present invention relates to a method for the detection and quantification of electro-inactive compounds in an Indicator Displacement Assay (IDA) by voltametric or amperometric detection using chemosensors comprising a metal-based indicator and a macrocyclic receptor, wherein the chemosensors are present in the test environment in the form of a solution or dispersion, which means the chemosensor is not bound or immobilized but can freely move or flow in the test sample. The method of the present invention is defined in claim 1.

As used herein, the terms "detection" or "analysis" in relation to the test method or assay described herein includes identification, i.e. qualitative measurement, as well as quantitative measurement, i.e. quantification tests.

The analytes to be evaluated with the method of the present invention belong to the group of so-called electro-inactive compounds, which are all analytes that do not show a pronounced electrochemical oxidation or reduction peak signal within the electrochemical window of water (in which the water is not electrolyzed). Generally, direct electrochemical detection of analytes that show a distinct oxidation or reduction current peak in the range of -0.9 - 1.2 V is generally possible (as can be seen with the many glucose sensors on the market). Any other analytes that show an oxidation or potential current peak outside this window (e.g., ammonium compounds) can no longer be detected directly by voltametric measurements in water. This is because beyond 1.2 V or -0.9 V, water decomposition (electrolysis) begins, making it impossible to detect a redox current of the analyte.

The electro-inactive analytes to be detected with the method of the present invention are preferably selected from biologically relevant analytes, including drug compounds like muscle relaxants, biomolecules, and antimicrobial agents. As a preferred class of electro-inactive analytes according to the invention quaternary ammonium compounds can be mentioned. This class of compounds is characterized by tetrasubstituted nitrogen that carries a pH-independent and thus permanent positive charge in aqueous solutions and biofluids. The presence of the permanent charge and the carbon-based backbone of this class of compounds provides a particularly high binding affinity for cucurbituril and pillararenes. For example, N,N,N-trimethyl-1-adamantylammonium hydroxide (TA) has a binding affinity of log Kₐ = 12.23 for CB7, while pancuronium bromide has a binding affinity of log Kₐ = 10.23. In comparison, amino acids (log K_{a,CB7} ≈ 2 - 5) or neurotransmitters such as serotonin (log K_{a,CB7} = 4.8) or dopamine (log K_{a,CB7} = 5.0) that might be present in body fluids like urine samples do not interfere in the detection of quaternary ammonium compounds like TA or pancuronium bromide.

Examples of quaternary ammonium compounds as target analytes according to the invention comprise N,N,N-trimethyl-1-adamantylammonium hydroxide (TA), pancuronium bromide, vecuronium bromide, scopolamine butylbromide (buscopan), acetylcholine, benzethonium chloride, oxyphenonium bromide, glycopyrronium bromide and cetyltrimethylammonium bromide. N,N,N-trimethyl-1-adamantylammonium hydroxide and the muscle relaxant pancuronium bromide are preferred target analytes. Some structures of preferred analytes are shown below:

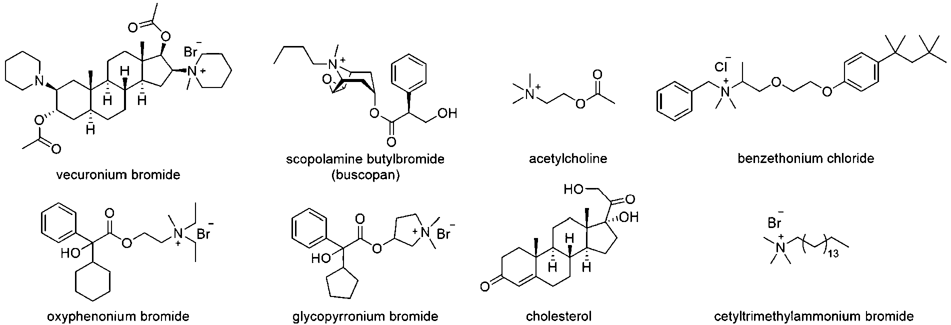

The term chemosensor as used herein is used in accordance with the general meaning of chemosensors as used in the analytical field. As used herein, the chemosensors are understood to refer to the combination of a receptor component and an indicator, which together allow to produce a recordable signal when getting in contact with the target analytes ("guest"). Accordingly, the chemosensors used herein comprise as the "receptors" ("host") macrocyclic molecules and as the "indicator" a metal-based compound. The two components can be but are not necessarily present in the form of a complex. It is also possible, to provide the two components separately to the test environment and allow self-assembling in vitro after addition to the sample.

The receptors of the chemosensors of the present invention are non-cyclodextrin-based receptors, which means that cyclodextrin-based chemosensors are excluded from the group of chemosensors according to the invention.

The chemosensors have a macrocyclic structure (like a ring) and therewith provide a cavity, into which smaller molecules (analytes or indicator / dye molecules) can penetrate. Therefore, such macrocyclic receptors can act as synthetic receptors by binding the analyte in the cavity. The macrocyclic receptors being suitable in the methods of the invention can be bi- or unimolecular chemosensors. In both cases, the cavity of the receptor can be filled with an indicator to perform the so-called "Indicator Displacement Assay", which is the underlying method for the detection of the target analytes of the present invention.

The macrocyclic receptors of the present invention must exhibit a suitable size of the cavities and suitable affinity to bind the target analytes. Suitable cavity sizes can be defined by a range of cavity diameter (d) of 5.8 < d ≤ 8.8 angstroms (Å), preferably of 6.5 < d ≤ 8.0 angstroms (Å). For example, the cavity size of CB7 is 7.3 Å.

Moreover, the macrocyclic receptors of the present invention must have a high binding affinity to the target analytes, which is achieved due to the hydrophobic effect and various noncovalent interactions with the charged portals as well as the non-charged "inner walls" of the cavity.

Preferably, the macrocyclic receptors of the chemosensors are selected from cucurbit*[n]*urils and pillar*[n]*arenes and derivatives thereof.

The group of cucurbit*[n]*urils relates to cucurbiturils in the sense of their general meaning and describes macrocyclic molecules made of glycoluril (=C₄H₂N₄O₂=) monomers linked by methylene bridges (-CH₂-). The oxygen atoms are located along the edges of the band and are tilted inwards, forming a partly enclosed cavity (cavitand). Cucurbiturils are commonly written as cucurbit*[n]*uril, wherein n is the number of glycoluril units. Common abbreviations are CB*[n]*, or simply CBn, which may also be used herein. Examples in accordance with the invention comprise cucurbituril-homologues with n=5 to 14, such as CB5, CB6, CB7, CB8, CB10, and CB14, among which the most preferred cucurbituril is CB7:

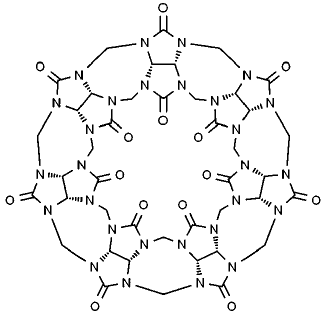

The group of pillararenes relates to pillararenes in the sense of their general meaning and describes macrocyclic molecules made of hydroquinone or dialkoxybenzene units (5 to 10) linked in the para position by methylene bridges. They are structurally similar to the cucurbiturils. In the case of sulfonated pillararenes, a strong electrostatic attraction between positively charged analytes and the negatively charged sulphone groups at the portals exist. Examples in accordance with the invention comprise pillar*[n]*arene-homologues with n=5 to 10 as well as 1,4-dimethoxypillar[5]aren and sulfonated pillar*[n]*arenes (n=5 to 10), such as in particular sulfonated pillar[5]arene (P5AS):

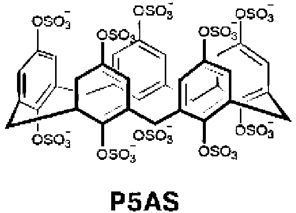

In a preferred aspect of the invention the macrocyclic receptors from the group of cucurbit*[n]*uril are selected from cucurbit[7]uril (CB7) and macrocyclic receptors from the group of pillar*[n]*arenes are selected from sulfonated pillar[5]arene (P5AS).

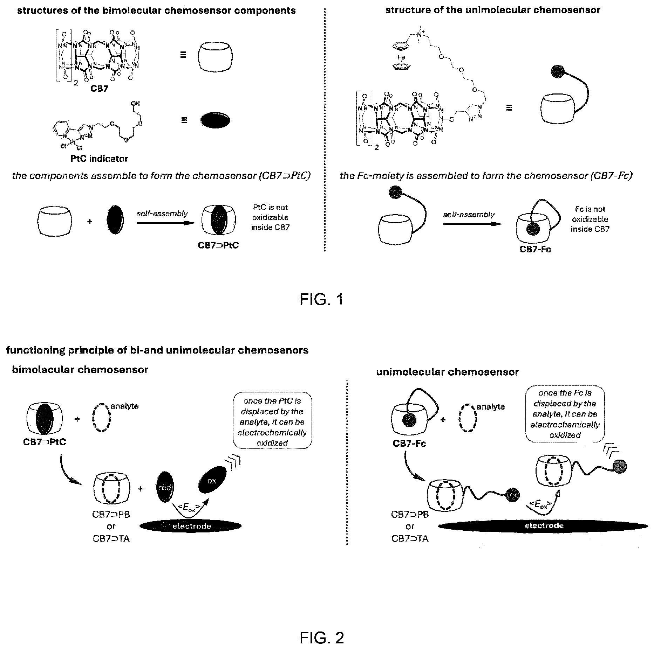

The chemosensors further comprise an indicator (also called "dye"). A suitable indicator for the method of the present invention must be able to self-assemble with the macrocyclic receptor or form a complex, as illustrated in Figures 1, 2 and 3. Further, the indicators must offer modulated electrochemical properties when they are bound inside the cavity of the receptors. However, as soon as they are displaced from the cavity, the electrochemical properties are restored. Thus, when the indicator is inside the cavity of the macrocyclic receptor, they are protected from electrochemical oxidation, which is reflected in the lower current measured when a positive electrode potential is applied. In contrast, when the indicators are displaced from the cavity of the receptors by the analytes, they can be easily oxidized and measured.

The indicators used in the present invention are metal-based indicators. Preferably, metal-based indicators are selected from platinum- or iron-based indicators. For iron-based indicators, the cyclopentadienyl iron complexes, also called ferrocene complexes, are the most suitable class of compounds. For platinum-based indicators, one could in principle also use other small Pt(II) complexes such as cis-platinum, carboplatin, oxaliplatin, or derivatives of the platinum complexes used here that carry other pyridinyl triazole ligands.

A particularly preferred platinum-based indicator is ((2-(2-(2-{4-(pyridin-2-yl)-1H-1,2,3-triazol-1-yl)eth-oxy)ethoxy)ethan-l-ol)dichloroplatin(II) (PtC). Such indicator forms a complex with the macrocyclic structure to provide a preferred chemosensor of the invention. Such preferred chemosensor can be obtained by mixing the receptor like cucurbituril, e.g. CB7, and the indicator PtC in equimolar amounts leading to the formation of an inclusion complex (CB7⊃PtC), wherein PtC resides inside the cavity of the receptor (e.g. CB7). CB7⊃PtC is a bimolecular chemosensor.

A particularly preferred iron-based indicator is 6-(ferrocenyl)-N,N,N-tirmethylethaneammonium (Fc). Such indicator binds to the macrocyclic structure to provide a preferred chemosensor of the invention. Such preferred chemosensor can be obtained by mixing the receptor like cucurbituril, e.g. CB7, and the indicator FC in equimolar amounts and allowing Fc to bind to the receptor to form a unimolecular chemosensor (CB7-FC). When the chemosensor self-assembles the FC enters the receptor cavity wherein Fc is not oxidized.

Particularly preferred chemosensors used in the method of the present invention are selected from platinum-cucurbit*[n]*uril, ferrocene-cucurbit*[n]*uril and ferrocene-P5AS, in particular from platinum-cucurbit[7]uril, ferrocene-cucurbit[7]uril and ferrocene-P5AS.

The method of the present invention is based on the principles of the so-called Indicator Displacement Assay (IDA). The IDA method is a well-known method based on the principle of a competitive displacement reaction of the bound indicator from the receptor by the analyte, whereby this displacement reaction can be analytically determined and followed by suitable measurement methods. In the IDA the analytes change the intensity of a spectroscopic signal. In the method of the present invention, two modifications of the general principle of the IDA methods are applied, using either bimolecular or unimolecular chemosensors as described below in more detail. The two modifications, underlying the present invention, are illustrated in Figures 1, 2 and 3. In both the electrochemical oxidation occurs after displacement of the indicator by the analyte, which can then be detected.

The new bi- and unimolecular chemosensors according to the present invention, using cucurbit*[n]*urils and pillararenes as the macrocyclic receptor are surprisingly suitable to overcome the limitations of cyclodextrin-based IDAs with voltammetric signal readout.

Positively charged analytes (guests), like quaternary ammonium compounds, provide a higher entropic binding contribution to the overall binding affinity resulting from the desolvation of the guest molecule upon binding to the receptors, while non-flat guests favor the release of energetic water (hydrophobic effect). This makes it a better macrocyclic receptor than cyclodextrins when detecting analytes at biologically relevant concentrations.

For example, quaternary ammonium compounds such as pancuronium bromide (PB) or N,N,N-trimethyl-adamantylammonium hydroxide (TA) have a higher binding affinity to CB7 than the electrochemical indicators PtC or the Fc moiety in CB7-Fc. As a result, their presence leads to the displacement of the indicator from the receptor cavity and the amount of indicator displaced is determined electrochemically via voltammetry or amperometry and can be correlated to the original amount of analyte present in the solution.

A preferred bimolecular chemosensor according to the invention comprises CB7 and the platinum-based indicator ((2-(2-(2-{4-(pyridin-2-yl)-1H-1,2,3-triazol-1-yl)eth-oxy)ethoxy)ethan-I-ol)dichloroplatin(II) (PtC) in the form of a complex. Such complex can be obtained by mixing CB7 and PtC in equimolar amounts leading to the formation of an inclusion complex (CB7⊃PtC), wherein PtC resides inside the cavity of the CB7. A preferred unimolecular chemosensor comprises CB7 and the iron-based indicator 6-(ferrocenyl)-N,N,N-tirmethylethaneammonium (Fc), which form a covalent conjugation of the Fc moiety to the rim of the macrocyclic host, yielding the CB7-Fc unimolecular chemosensor.

These particularly preferred chemosensors surprisingly allow to bind electro-inactive target analytes to the macrocyclic receptor in water, buffer, and urine samples. In addition, these chemosensors turned out to remain stable and do not precipitate in the solution.

Accordingly, in the method of the invention the detection and quantification of the analytes can be carried out in an aqueous medium, comprising aqueous test samples, buffer solutions or biofluids, including water, physiological saline, PBS, artificial CSF, urine, saliva, blood (comprising blood serum/human serum (HS) and human serum albumin/human serum albumin (HSA)), CSF, amniotic fluid, semen and sweat.

Preferably, the chemosensors are directly assembled in the aqueous medium of the test sample, which means that no immobilization on a substrate, surface or electrode is necessary. That means, the chemosensors are present in the test environment in solution, which should be understood to be free-flowing or free-moving or dispersed in the test solution (in contrast to being immobilized on any surface).

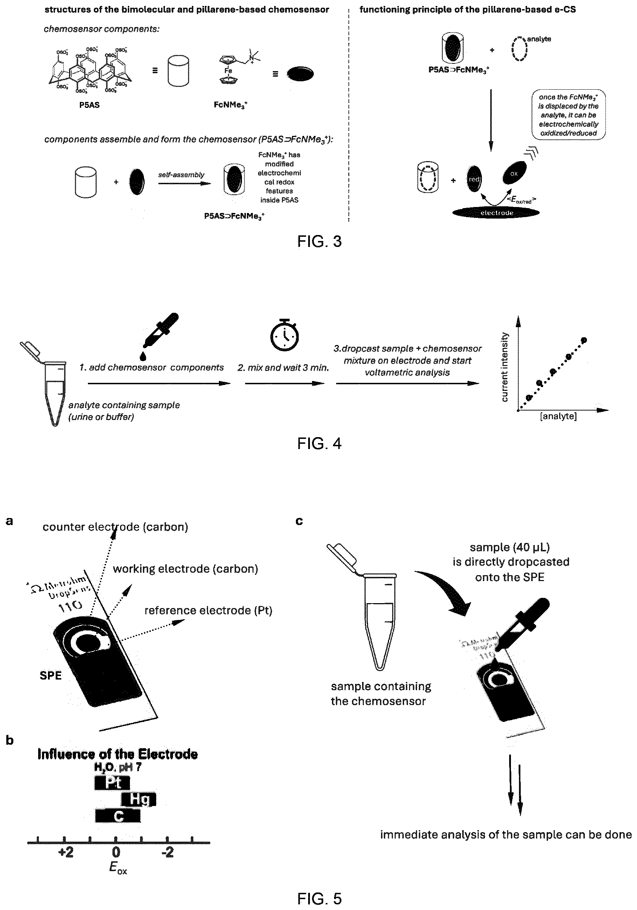

This provides a particular advantage of the invention. Instead of immobilizing the IDA-based chemosensors on the surface of electrodes or other components of the device the new assay developed by the inventors of the present invention allows to add the self-assembling chemosensors directly in the test samples, like biofluids or aqueous samples. After a short equilibration time, the sample-chemosensor mixture can be directly drop-casted onto commercially available screen-printed electrodes (SPEs) for voltammetric detection. This improved testing scheme is illustrated in Figure 4.

Such simplified new assay protocol is enabled by the use of the new uni- and bimolecular chemosensors of the invention, which can be applied directly to the test sample, e.g. by adding each sensor component in the form of a solution stepwise to the test sample containing the analyte, where the chemosensor then self-assembles. Such simplified test protocol comprises adding the macrocyclic host to the test sample comprising the target analyte, followed by adding the metal-based indicator (or vice versa). After dropping the chemosensor-sample-mixture onto the electrode, it can be analysed by voltammetric measurements. The total analysis time is short and can be a few minutes, such as < 5 minutes, preferably < 3 minutes. Alternatively, the already self-assembled chemosensor as such can be added to the test solution containing the analyte.

For example, when using the preferred CB7⊃PtC bimolecular chemosensor, the assay is preferably performed by stepwise addition of an excess of a CB7 to the analyte sample followed by equimolar addition of a PtC solution. After a short equilibration time, a minimal amount of the resulting chemosensor-analyte-sample mixture is placed onto a commercially available carbon SPE, followed by voltammetric or amperometric measurements to obtain current-response curves, which are indicative of the analyte and can be used for quantification of the analyte. Minimal sample pre-treatment is required, i.e., simple dilution with buffers instead of otherwise complicated separation or extraction protocols.

As mentioned above, as a further particular advantage the present invention provides a chemosensor that is compatible with commercially available carbon SPEs. The use of carbon as an electrode material allows to apply oxidation Potentials up to 1.2 V in aqueous media and biofluids while providing an electrode surface that does not interact with the chemosensors. This is possible because the chemosensor remains stable in the solution, as there are no major attractive forces that can cause the adsorption of the chemosensor onto the surface of the SPE. The assay methods described herein use water-soluble and stable chemosensors. The solution to be analysed can then be dropped onto the surface of commercially available SPEs, requiring only a minimal sample volume and the analyte is then detected by applying an oxidative potential to the SPE working electrode, and the analyte can be detected by amperometric or various voltammetric methods. Such a simplified test protocol is illustrated in Figure 5.

Accordingly, in further preferred aspects in the method of the invention the voltametric or amperometric detection is carried out using a screen-printed electrode (SPE). Further, preferably, the chemosensor is applied directly to such a screen-printed electrode (SPE).

It is further possible, that the method of the invention as described herein is carried out without sample preparation or pre-treatment, such as preparatory addition of co-solvents, purification and/or separation steps.

A further aspect of the invention relates to a chemosensor device or diagnostic kit for the detection and quantification of biologically relevant analytes, which comprises a chemosensor as defined herein, namely a chemosensor comprising a metal-based indicator and a macrocyclic receptor, and at least one screen-printed electrode (SPE), and an electrochemical signal readout operating via voltammetry or amperometry. In such a device or kit, the chemosensor can be present in the form of an already assembled complex or in a separated spatial arrangement of the receptor and indicator components to be added to the test sample separately (as described above).

In such chemosensor device or diagnostic kit the chemosensor and its receptor and indicator components are characterized as defined above. The method of the present invention is defined in claim 10.

In a preferred embodiment, such a chemosensor device or diagnostic kit for the detection and quantification of biologically relevant analytes as defined herein is a miniaturized portable and disposable device.

A further aspect of the invention relates to chemosensors comprising a metal-based indicator and a macrocyclic receptor as described herein, such as preferably chemosensors, which are selected from platinum-cucurbit[7]uril, ferrocene-cucurbit[7]uril and ferrocene-P5AS.

A further aspect of the invention relates to the use of the chemosensors as described herein for the voltametric or amperometric detection and quantification of electro-inactive compounds, in particular such as defined herein, in an Indicator Displacement Assay (IDA).

A further aspect of the invention relates to the use of the chemosensor device or diagnostic kit as described herein as ready-to-use sensors at the point of care, comprising home-use, clinic-use, use in crime scenes and law enforcement and use as wearable (portable) sensors.

A very particular aspect of the invention relates to the use of the method described herein in the detection of drug abuse.

The invention is illustrated further in the Examples and Figures below.

### DESCRIPTION OF THE FIGURES

- Fig. 1: Illustration of the IDA functioning principle using CB7 as a cucurbituril-based macrocyclic receptor showing the unimolecular design with ferrocene as the indicator and the bimolecular design with a Pt-based indicator.
- Fig. 2: Additional illustration of the IDA functioning principle using CB7 as a cucurbituril-based macrocyclic receptor showing the unimolecular design with ferrocene as the indicator and the bimolecular design with a Pt-based indicator.
- Fig. 3: Illustration of the IDA functioning principle using P5AS as a pillararene-based macrocyclic receptor with a ferrocene-based indicator.
- Fig. 4: Illustration of the test protocol using self-assembling chemosensors of the invention.
- Fig. 5: a) Illustration of a commercial SPE used in a method according to the invention for the detection of quaternary ammonium compounds. b) Effect of electrode material (working and counter electrode) on the electrochemical window when neutral pH water is used. c) Illustration of a test protocol comprising adding the chemosensor components to a sample, showing the required minimal amounts to be added onto the SPE.
- Fig. 6: a) Cyclic voltammetry studies of PtC (200 µM) and CB7⊃PtC (200 µM) in water at pH 7.0 (scan rate: 50 mV·s⁻¹). b) TA-dependent *i*_{pc} (at 0.9 V) obtained from CV experiments in water at pH 7.0; [CB7⊃PtC] = 200 µM; scan rate: 50 mV·s⁻¹.
- Fig. 7: a) Cyclic voltammetry studies of CB7-Fc (200 µM) in the presence or absence of TA (400 µM) in saline solution (0.1 M NaCl) at pH 7.0 (scan rate: 100 mV·s⁻¹). b) The current vs. scan rate plot was obtained with CB7-Fc in saline solution (0.1 M NaCl) at pH 7.0 (scan rate: 100 mV·s⁻¹).
- Fig. 8: a) PB-dependent *i*ₒₓ obtained from solutions of PB in PBS (5 mM, pH 7.0) with CB7⊃PtC (50 µM), reported is the observed current *i*ₒₓ at 50 s (Eₒₓ = 0.9 V). b) SCV-based detection of PB in human urine (1: 3 diluted in 5 mM PBS at pH 7.0, [CB7⊃PtC] = 50 µM), reported is the observed current *i*ₒₓ at 0.9 V. The average *i*ₒₓ and the corresponding standard deviation (δ) were calculated from three independent measurements.
- Fig. 9: Electrochemical properties of a pillararene-based chemosensor and the electrochemical detection of TA; [P5AS] = 50 µM, [FcNMe³⁺] = 50 µM; measurements performed in PBS (5 mM, pH7).

### EXAMPLES

The invention is described further by the following examples, without being limited thereto.

### 1. Cucurbituril (CB7) based Chemosensors

### 1.1 Bimolecular chemosensor (CB7⊃PtC)

A bimolecular chemosensor with CB7 as the receptor and PtC as the Pt-based indicator can be prepared as follows:

### a) Preparation of PtC

The Pt-based indicator PtC was prepared through a copper-catalyzed azide-alkyne cycloaddition reaction between 2-ethynylpyridine and (2-(2-(2-(2-az-idoethoxy)ethoxy)ethan-1-ol) (2) in 62 % yield [Moreno-Alcantar, G. et al., Solvent-Driven Supramolecular Wrapping of Self-Assembled Structures, Angew. Chem. Int. Ed. 2021, 60 (10), 5407-5413*.]*

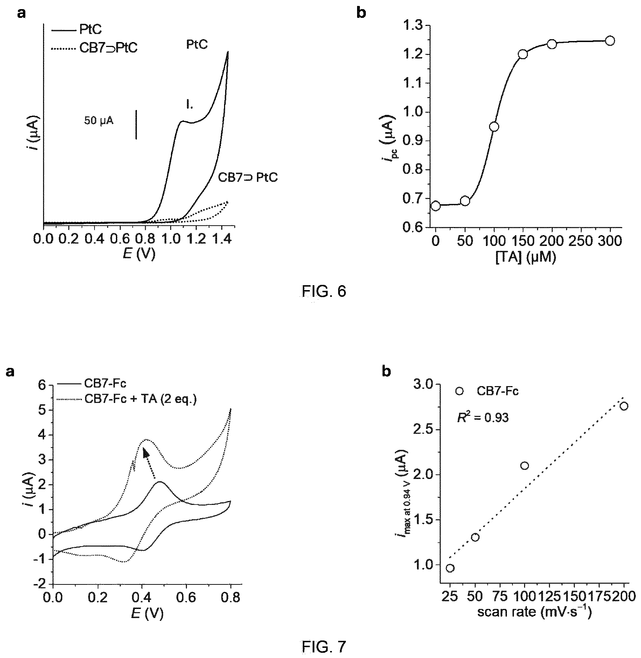

The subsequent complexation using cis-dichlorobis(dimethyl-sulfoxide)platinum(II) as the Pt(ll) source gave the final PtC in a very good yield (87 %). CV studies further support the formation of the CB7⊃PtC inclusion complex (see Figure 6a).

The intermediate binding affinity of PtC is advantageous for its use in displacement assays for the detection of bioanalytes.

The analysis of the test analyte was carried out following the test protocol as illustrated in Figure 4 and 5.

In the case of the bimolecular chemosensor (CB7⊃PtC), a negligibie current (*i*ₒₓ at Eₒₓ = 0.9 V) is observed in the absence of the analyte in solution because the indicator is not oxidizable as shown in Figure 6a by comparing with the current observed for free PtC. However, an increasing current is observed when increasing amounts of the analyte TA are present in the sample (Figure 6b).

### 1.2 Unimolecular chemosensor (CB7-Fc)

A unimolecular chemosensor with CB7 as the receptor and Fc as the iron-based (ferrocene-based) indicator can be prepared as follows:
In a first step, (dimethylaminomethyl)ferrocene was alkylated with di-bromo-1-bromo-2-(2-(2-(2-bromoethoxy)ethoxy)ethane (Br-TEG-Br) to give the N-(2-(2-(2-bromoethoxy)ethoxy)ethyl)-N,N-dimethylferrocenylmethylammonium derivative (Fc-TEG-Br) in 70% yield. Subsequent reaction of Fc-TEG-Br with sodium azide afforded the N-(2-(2-(2-(2-azidoethoxy)ethoxy)ethoxy)ethyl)-N,N-dimethylferrocenylmethylammonium intermediate (, Fc-TEG-N₃; 48% yield), which was used to covalently link it to the mono-propargyl cucurbit[7]uril via a copper-catalyzed azide-alkyne cycloaddition *[*Hu, C. et al. Covalent cucurbit[7]uril-dye conjugates for sensing in aqueous saline media and biofluids Chem. Sci., 2020, 11, 11142-11153*].*

The analysis of the test analyte was carried out following the test protocol as illustrated in Figure 4 and 5.

For the unimolecular chemosensor, the behavior is the same as for CB7⊃PtC. In the absence of the analyte, the current intensity is significantly reduced. However, upon addition of the analyte, the current intensity increases (Figure 7a) as the indicator is displaced, facilitating its oxidation. Further, a significant shift in the oxidation potential for the displaced indicator can be observed, indicating that oxidation may occur at lower potentials. Thus, with these observed changes, the detection of quaternary ammonium compounds is possible.

Performing cyclic voltammetry at different scanning rates (Figure 7b) shows that the chemosensors do not deposit on the electrode or aggregate, as the curves obtained show a linear relationship between sampling rate and peak intensity.

### 1.3 Detection of pancuronium bromide as the target analyte

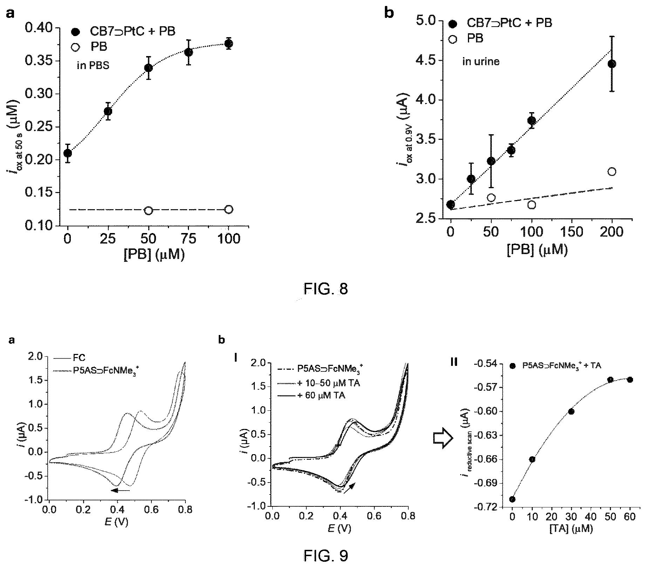

The muscle relaxant pancuronium bromide (PB) can be detected in buffer and urine samples at biorelevant concentrations according to the principles described above and illustrated in Figure 2. Detection can be performed in the amperometric mode (Figure 8a) or by voltammetry, e.g., staircase voltammetry (SCV in Figure 8b). In the amperometric mode (in PBS), a detection limit of 17.7 pM was calculated, whereas when PB was detected in urine by SCV, the detection limit was 7.6 pM. The lower detection limit by SCV is due to the reduction of capacitive charges by this detection method, which is achieved by applying a series of regular potential pulses superimposed on the potential Step ramp. Therefore, SCV-based measurements are common in biomedical diagnostics.

The analysis of the test analyte was carried out following the test protocol as illustrated in Figure 4 and 5.

### 2. Pillararene (P5AS) based Chemosensor

A chemosensor with P5AS as the receptor and an iron-based (ferrocene-based) indicator can be prepared as follows: The sulfonated pillararene (P5AS) was prepared in a three-step synthesis. First, the fully methylated OMe-pillar[5]arene was synthesized by the reaction of 1,4-dimethoxybenzene and paraformaldehyde in the presence of boron trifluoride etherate. The OMe-pillar[5]aren was precipitated by introducing the reaction mixture into methanol and purified by column chromatography (yield = 70%). In the subsequent reaction step, the OMe-pillar[5]aren was de-methoxylated upon reaction with tribromide to give the fully hydroxylated pillar[5]aren (HO- pillar[5]aren). The HO-pillar[5]aren was precipitated by pouring the reaction mixture into methanol, collected by filtration, and purified by column chromatography (yield = 70%). Sulfonation of the HO-pillar[5]arene with the pyridine-sulfur trioxide complex in the presence of pyridine gave the fully sulfonated P5AS after its purification by precipitation in EtOH.

The iron-based (ferrocene-based) indicator was prepared by methylation of (dimethylaminomethyl)ferrocene with methyl iodide.

The structures and operating principle of the P5AS-based electrochemical chemosensor are shown in Figure 3. The operating principle is the same as described above for a bimolecular chemosensor. The detection of quaternary ammonium compounds (e.g. drugs or metabolites) becomes possible because the ferrocene-based electrochemical indicator forms an inclusion complex with the P5AS (P5AS⊃FcNMe³⁺). In this complexed state, the electrochemical properties of the indicator change so that its oxidation and reduction are shifted to lower potentials as was assessed by cyclic voltammetry (Figure 9a). However, in the presence of N,N,N-trimethyl-1-adamantylammonium hydroxide (TA) as the target analyte, the indicator is displaced from the macrocycle, which in turn can be detected by a significant change in the current in the reduction scan (Figure 9b I). Specifically, the presence of TA is reflected in a concentration-dependent decrease in current in the branch of the reduction curve (at E = 0.39 V; Figure 9b II)).

### 3. Methods

### Electrochemical measurements:

CV, chronoamperometric, and DPV measurements were performed using a Metrohm portable bipotentiostat/galvanostat (± 4 V potential range, ± 40 mA maximum measurable current) (SpectroECL). The electrochemical setup is controlled by DropView SPELEC software. Screen-printed carbon electrodes (SPEs; DRP-110 from Metrohm DropSense) were used for the measurements.

## Claims

1. A method for the detection and quantification of electro-inactive compounds in an Indicator Displacement Assay (IDA) by voltametric or amperometric detection using chemosensors comprising a metal-based indicator and a macrocyclic receptor which is a non-cyclodextrin-based receptor selected from cucurbit*[n]*uril and pillar*[n]*arenes and derivatives thereof, and wherein the chemosensors are present in the test environment in form of a solution or dispersion.

2. The method according to claim [1], wherein the electro-inactive compounds are selected from biologically relevant analytes, including drug compounds like muscle relaxants, biomolecules, and antimicrobial agents, and which are quaternary ammonium compounds, including N,N,N-trimethyl-1-adamantylammonium hydroxide, pancuronium bromide, vecuronium bromide, scopolamine butylbromide (buscopan), acetylcholine, benzethonium chloride, oxyphenonium bromide, glycopyrronium bromide, and cetyltrimethylammonium bromide.

3. The method according to claim [1] or [2], wherein the chemosensors are bi- or unimolecular chemosensors.

4. The method according to any one of claims [1] to [3], wherein macrocyclic receptors from the group of cucurbit*[n]*uril are selected from cucurbit[7]uril (CB7) and macrocyclic receptors from the group of pillar*[n]*arenes are selected from sulfonated pillararen[5]arene (P5AS).

5. The method according to any one of claims [1] to [4], wherein metal-based indicators of the chemosensors are selected from platinum- or iron-based indicators.

6. The method according to any one of claims [1] to [5], wherein the chemosensors are selected from platinum-cucurbit[7]uril, ferrocene-cucurbit[7]uril and ferrocene-P5AS.

7. The method according to any one of claims [1] to [6], wherein the detection and quantification is carried out in an aqueous medium, comprising aqueous test samples, buffer solutions or biofluids, including water, physiological saline, PBS, artificial CSF, urine, saliva, blood (comprising blood serum/human serum (HS) and human serum albumin/human serum albumin (HSA)), CSF, amniotic fluid, semen and sweat.

8. The method according to any one of claims [1] to [7], wherein the voltametric or amperometric detection is carried out with screen-printed electrodes (SPEs).

9. The method according to any one of claims [1] to [8], comprising
a step of adding the macrocyclic receptor and the metal-based indicator separately to the aqueous medium of the test sample and allow self-assembling in vitro;
and/or
a step of dropping the chemosensor directly to a screen-printed electrode (SPE).

10. A chemosensor device or diagnostic kit for the detection and quantification of biologically relevant analytes, comprising
- a chemosensor comprising a metal-based indicator and a macrocyclic receptor selected from cucurbit[n]uril and pillar[n]arenes and derivatives thereof in the form of a solution or dispersion as defined in one or more of claims [1] to [6],
- screen-printed electrodes (SPEs), and
- an electrochemical signal readout operating via voltammetry or amperometry.

11. The chemosensor device or diagnostic kit according to claim [10] for the detection and quantification of biologically relevant analytes, which is a miniaturized portable and disposable device.

12. Chemosensor comprising a metal-based indicator and a macrocyclic receptor selected from platinum-cucurbit[7]uril, ferrocene-cucurbit[7]uril and ferrocene-P5AS.

13. Chemosensor according to claim [12] which is provided in the form of a solution or dispersion.

14. Use of the chemosensor device or diagnostic kit according to claims [10] or [11] or the chemosensor according to claims [12] or [13] for the voltametric or amperometric detection and quantification of electro-inactive compounds in an Indicator Displacement Assay (IDA), preferably for the detection of drug abuse.

15. Use of the chemosensor device or diagnostic kit according to claims [10] or [11] as ready-to-use sensors at the point of care, comprising home-use, clinic-use, use in crime scenes and law enforcement and use as wearable sensors.

## Patentansprüche

1. Ein Verfahren zum Nachweis und zur Quantifizierung von elektroinaktiven Verbindungen in einem Indikator-Verdrängungs-Assay (IDA) durch voltametrischen und amperometrischen Nachweis mithilfe von Chemosensoren umfassend einen metallbasierten Indikator und einen makrozyklischen Rezeptor welcher ein nicht-Cyclodextrin-basierter Rezeptor ausgewählt aus Cucurbit[*n*]Urilen und Pillar[*n*]Arenen und Derivaten davon ist, und worin die Chemosensoren in der Testumgebung in Form einer Lösung oder Dispersion vorhanden sind.

2. Das Verfahren gemäß Anspruch [1] worin elektroinaktive Verbindungen ausgewählt werden aus biologisch relevanten Analyten, einschließlich Arzneimittelwirkstoffen wie Muskelrelaxanzien, Biomolekülen und antimikrobiellen Wirkstoffen, und bei denen es sich um quaternäre Ammoniumverbindungen handelt, einschließlich N,N,N-trimethyl-1-adamantylammoniumhydroxid, Pancuroniumbromid, Vecuroniumbromid, Scopolambutylbromid (Buscopan), Acetylcholin, Benzethoniumchlorid, Oxyphenoniumbromid, Glycopyrroniumbromid und Cetyltrimethylammoniumbromid.

3. Das Verfahren gemäß Anspruch [1] oder [2], worin die Chemosensoren bi- oder unimolekulare Chemosensoren sind.

4. Das Verfahren gemäß einem von Anspruch [1] bis [3], worin makrozyklische Rezeptoren aus der Gruppe der Cucurbit[*n*]Urile ausgewählt sind aus Cucurbit[7]Uril (CB7) und makrozyklische Rezeptoren aus der Gruppe der Pillar[*n*]Arene ausgewählt sind aus sulfoniertem Pillararen[5]Aren (P5AS).

5. Das Verfahren gemäß einem von Anspruch [1] bis [4], worin metallbasierte Indikatoren der Chemosensoren ausgewählt sind aus Platin- oder Eisen-basierten Indikatoren.

6. Das Verfahren gemäß einem von Anspruch [1] bis [5], worin die Chemosensoren ausgewählt sind aus Platin-Cucurbit[7]Uril, Ferrocen-Cucurbit[7]Uril und Ferrocen-P5AS.

7. Das Verfahren gemäß einem von Anspruch [1] bis [6], worin der Nachweis und die Quantifizierung in einem wässrigen Medium ausgeführt werden, das wässrige Testproben, Pufferlösungen oder Biofluide umfasst, einschließlich Wasser, physiologische Kochsalzlösung, PBS, künstliches CSF, Urin, Speichel, Blut (umfassend Blutserum/humanes Serum (HS) und humanes Serumalbumin/humanes Serumalbumin (HAS)), CSF, Fruchtwasser, Samen und Schweiß.

8. Das Verfahren gemäß einem von Anspruch [1] bis [7], worin der voltametrische oder amperometrische Nachweis mit siebgedruckten Elektroden (SPEs) ausgeführt wird.

9. Das Verfahren gemäß einem von Anspruch [1] bis [8], umfassend
einen Schritt in welchem der makrozyklische Rezeptor und der metallbasierte Indikator getrennt voneinander zu dem wässrigen Medium der Testprobe hinzugegeben werden und sich in-vitro selbst zusammenlagern;
und/oder
einen Schritt bei welchem der Chemosensor direkt auf die siebgedruckte Elektrode (SPE) getropft wird.

10. Eine Chemosensorvorrichtung oder ein Diagnosekit zum Nachweis und zur Quantifizierung biologisch relevanter Analyten, umfassend
- einen Chemosensor umfassend einen metallbasierten Indikator und einen makrozyklischen Rezeptor ausgewählt aus Cucurbit[*n*]Urilen und Pillar[*n*]Arenen und Derivaten davon in Form einer Lösung oder Dispersion wie in einem oder mehreren der Ansprüche [1] bis [6] definiert,
- siebgedruckten Elektroden (SPEs), und
- eine elektrochemische Signalauslesung, die mittels Voltammetrie oder Amperometrie operiert.

11. Die Chemosensorvorrichtung oder das Diagnosekit gemäß Anspruch [10] zum Nachweis und zur Quantifizierung biologisch relevanter Analyten, bei welcher es sich um eine miniaturisierte, tragbare und wegwerfbare Vorrichtung handelt.

12. Chemosensor umfassend einen metallbasierten Indikator und einen makrozyklischen Rezeptor ausgewählt aus Platin-Cucurbit[7]Uril, Ferrocen-Cucurbit[7]Uril und Ferrocen-P5AS.

13. Chemosensor gemäß Anspruch [12], welcher in Form einer Lösung oder Dispersion zur Verfügung gestellt wird.

14. Verwendung der Chemosensorvorrichtung oder des Diagnosekits gemäß Anspruch [10] oder [11] oder des Chemosensors gemäß Anspruch [12] oder [13] für den voltametrischen oder amperometrischen Nachweis von elektroinaktiven Verbindungen in einem Indikator-Verdrängungs-Assay (IDA), bevorzugt für den Nachweis von Arzneimittelmissbrauch.

15. Verwendung der Chemosensorvorrichtung oder des Diagnosekits gemäß Anspruch [10] oder [11] als gebrauchsfertige Sensoren am Versorgungsort, einschließlich der Verwendung zu Hause, in Kliniken, an Tatorten und bei der Strafverfolgung sowie als tragbare Sensoren.

## Revendications

1. Procédé de détection et de quantification de composés électro-inactifs dans un test de déplacement d'indicateur (IDA) par détection voltamétrique ou ampérométrique à l'aide de chimiosenseurs comprenant un indicateur à base de métal et un récepteur macrocyclique qui est un récepteur non à base de cyclodextrine choisi parmi le cucurbit[*n*]uril et les pillar[*n*]arènes et leurs dérivés, et dans lequel les chimiosenseurs sont présents dans l'environnement de test sous la forme d' , d'une solution ou d'une dispersion .

2. Procédé selon la revendication [1], dans lequel les composés électro-inactifs sont choisis parmi des analytes biologiquement pertinents, y compris des composés médicamenteux tels que des myorelaxants, des biomolécules et des agents antimicrobiens, et qui sont des composés d'ammonium quaternaire, y compris l'hydroxyde de N,N,N-triméthyl-1-adamantylammonium hydroxyde, le bromure de pancuronium, le bromure de vécuronium, le bromure de butylscopolamine (Buscopan), l'acétylcholine, le chlorure de benzéthonium, le bromure d'oxyphénonium, le bromure de glycopyrronium et le bromure de cétyltriméthylammonium.

3. Procédé selon la revendication [1] ou [2], dans lequel les chimiosenseurs sont des chimiosenseurs bi- ou unimoleculaires.

4. Procédé selon l'une quelconque des revendications [1] à [3], dans lequel les récepteurs macrocycliques du groupe des cucurbit[*n*]uriles sont choisis parmi le cucurbit[7]uril (CB7) et les récepteurs macrocycliques du groupe des pillar[*n*]arènes sont choisis parmi le pillararène[5]arène sulfoné (P5AS).

5. Procédé selon l'une quelconque des revendications [1] à [4], dans lequel les indicateurs à base de métal des capteurs chimiques sont choisis parmi les indicateurs à base de platine ou de fer.

6. Procédé selon l'une quelconque des revendications [1] à [5], dans lequel les chimiosenseurs sont choisis parmi le platine-cucurbit[7]uril, le ferrocène-cucurbit[7]uril et le ferrocène-P5AS.

7. Procédé selon l'une quelconque des revendications [1] à [6], dans lequel la détection et la quantification sont effectuées dans un milieu aqueux, comprenant des échantillons de test aqueux, des solutions tampons ou des biofluides, y compris de l'eau, une solution saline physiologique, du PBS, du LCR artificiel, de l'urine, de la salive, du sang (comprenant du sérum sanguin/sérum humain (HS) et de l'albumine sérique humaine/albumine sérique humaine (HSA)), du LCR, du liquide amniotique, du sperme et de la sueur.

8. Procédé selon l'une quelconque des revendications [1] à [7], dans lequel la détection voltamétrique ou ampérométrique est effectuée à l'aide d'électrodes sérigraphiées (SPE).

9. Procédé selon l'une quelconque des revendications [1] à [8], comprenant
une étape consistant à ajouter séparément le récepteur macrocyclique et l'indicateur à base de métal au milieu aqueux de l'échantillon à tester et à laisser s'opérer l'auto-assemblage in vitro ;
et/ou
une étape consistant à déposer le chimiosenseur directement sur une électrode sérigraphiée (SPE).

10. Dispositif de chimiosenseur ou kit de diagnostic pour la détection et la quantification d'analytes biologiquement pertinents, comprenant
- un capteur chimique comprenant un indicateur à base de métal et un récepteur macrocyclique choisi parmi le cucurbit[*n*]uril et les pillar[*n*]arènes et leurs dérivés sous la forme d'une solution ou d'une dispersion telle que définie dans l'une ou plusieurs des revendications [1] à [6],
- des électrodes sérigraphiées (SPE), et
- un système de lecture de signaux électrochimiques fonctionnant par voltamétrie ou ampérométrie.

11. Dispositif de chimiosensor ou kit de diagnostic selon la revendication [10] pour la détection et la quantification d'analytes biologiquement pertinents, qui est un dispositif miniaturisé, portable et jetable.

12. Capteur chimique comprenant un indicateur à base de métal et un récepteur macrocyclique choisi parmi le platine-cucurbit[7]uril, le ferrocène-cucurbit[7]uril et le ferrocène-P5AS.

13. Capteur chimique selon la revendication [12], qui se présente sous la forme d'une solution ou d'une dispersion.

14. Utilisation du dispositif de chimiosenseur ou du kit de diagnostic selon la revendication [10] ou [11] ou du chimiosenseur selon la revendication [12] ou [13] pour la détection et la quantification voltamétriques ou ampérométriques de composés électro-inactifs dans un test de déplacement d'indicateur (IDA), de préférence pour la détection de l'abus de drogues.

15. Utilisation du dispositif de chimiosenseur ou du kit de diagnostic selon la revendication [10] ou [11] en tant que capteurs prêts à l'emploi au point de service, comprenant une utilisation à domicile, en clinique, sur les scènes de crime et par les forces de l'ordre, ainsi qu'une utilisation en tant que capteurs portables.
